# DEMANDE DE BREVET EUROPEEN

(11) **EP 1 466 908 A1**
(43) Date de publication de la demande: **13.10.2004**
(21) Numéro de dépôt: 04290373.2
(22) Date de dépôt: 12.02.2004
(51) Int. Cl.: C07D 251/70, A61K 7/42, C08K 5/3492, C07D 251/52, C07D 251/48

(54) **Nouveaux dérivés de s-triazine possédant au moins un sel d'acide para-amino benzalmalonique greffé et compositions cosmétique photoprotectrices contenant ces dérivés**

(30) Priorité: 03.03.2003 FR 0302562
(71) Demandeur: L'OREAL, 75008 Paris (FR)
(72) Inventeur: Richard, Hervé, 93420 Villepinte (FR); Luppi, Bernadette, 77290 Mitry Mory (FR)
(74) Mandataire: Miszputen, Laurent

(57) **Abrégé**

L'invention concerne des compositions photoprotectrices, comprenant des dérivés de s-triazine de formule (1) possédant au moins un sel d'acide para-amino benzalmalonique greffé à titre de filtres solaires actifs dans le domaine des rayonnements UV. 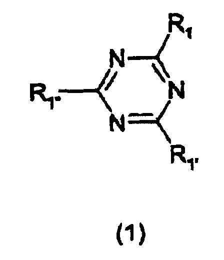 dans laquelle au moins un des radicaux R₁,R₁' et R₁", identiques ou différents, désigne un groupe chromophore répondant à la formule

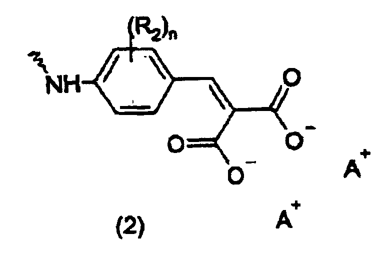

## Description

L'invention concerne des compositions photoprotectrices, comprenant des dérivés de s-triazine possédant au moins un sel d'acide para-amino benzalmalonique greffé à titre de filtres solaires actifs dans le domaine des rayonnements UV.

L'invention concerne également de nouveaux dérivés de s-triazine possédant au moins un sel d'acide para-amino benzalmalonique greffé et leurs utilisations.

On sait que les radiations de longueurs d'onde comprises entre 280 nm et 400 nm permettent le brunissement de l'épiderme humain, et que les radiations de longueurs d'onde comprises entre 280 et 320 nm connues sous la dénomination de radiations UV-B, provoquent des érythèmes et des brûlures cutanées qui peuvent nuire au développement du bronzage naturel. Pour ces raisons ainsi que pour des raisons esthétiques, il existe une demande croissante de moyens de contrôle de ce bronzage naturel. Il convient donc de filtrer ce rayonnement UV-B.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible et/ou continuellement exposée au rayonnement solaire. Les rayons UV-A entraînent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photoallergiques. Ainsi, pour des raisons esthétiques et cosmétiques telles que la conservation de l'élasticité naturelle de la peau, de plus en plus de personnes désirent contrôler l'effet des rayons UV-A sur leur peau. On entend par facteur de protection solaire le rapport du temps d'irradiation nécessaire pour atteindre le seuil érythématogène en présence du filtre testé au temps d'irradiation nécessaire pour atteindre ce même seuil en l'absence de filtre.

Il est donc souhaitable de disposer de composés susceptibles d'absorber aussi les rayons UV-B que les rayons UV-A.

Les filtres organiques sont le plus souvent formulés dans des compositions présentent sous forme d'émulsions huile-dans-eau ou eau-dans-huile Les filtres organiques, généralement lipophiles ou hydrophiles, sont présents sous forme dissoute, dans l'une ou l'autre de ces phases, en des quantités appropriées pour obtenir le facteur de protection solaire (FPS) recherché.

On entend par facteur de protection solaire le rapport du temps d'irradiation nécessaire pour atteindre le seuil érythématogène en présence du filtre testé au temps d'irradiation nécessaire pour atteindre ce même seuil en l'absence de filtre.

Outre leur pouvoir filtrant du rayonnement solaire, les composés photoprotecteurs doivent également présenter de bonnes propriétés cosmétiques, une bonne solubilité dans les solvants usuels et notamment dans les milieux aqueux et une photostabilité satisfaisante.

La demanderesse a découvert de manière surprenante une nouvelle famille de dérivés de s-triazine hydrosolubles possédant au moins un sel d'acide para-amino benzalmalonique greffé présentant des propriétés d'absorption du rayonnement UV dans la zone des radiations UV-B et dans la zone des UV-A courts. Ces composés peuvent être incorporés dans des formulations cosmétiques. Ils présentent une bonne solubilité dans les milieux aqueux, une bonne photostabilité et présentent des qualités cosmétiques satisfaisantes.

L'un des objets de la présente invention, consiste en de nouveaux composés dérivés de s-triazine hydrosolubles possédant au moins un sel d'acide para-amino benzalmalonique greffé répondant à la formule suivante (1) que l'on définira plus en détail par la suite.

L'invention concerne également une composition cosmétique ou dermatologique, destinée à la photoprotection des matières kératiniques, contenant dans un milieu cosmétiquement acceptable au moins un composé de formule (1).

D'autres objets apparaîtront à la lumière de la description.

Les composés conformes à la présente invention répondent à la formule générale (1) suivante : 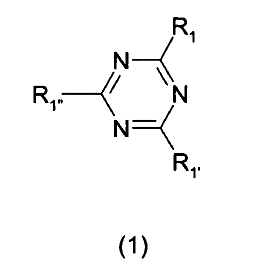 dans laquelle :
R₁, R₁' et R₁'', identiques ou différents, désignent :
   (i) soit un groupe chromophore répondant à l'une des formules suivantes : 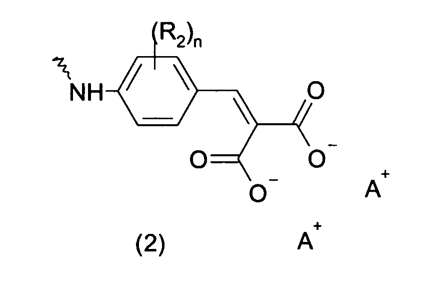 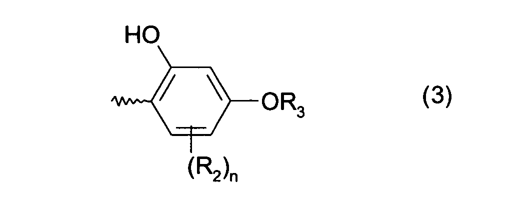 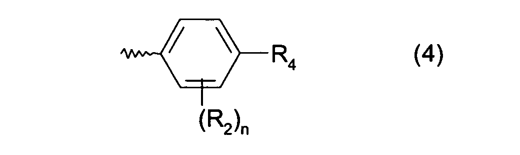 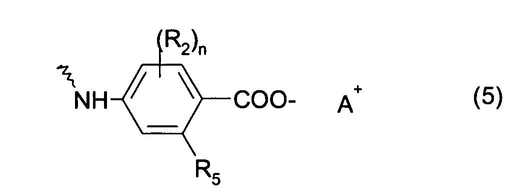 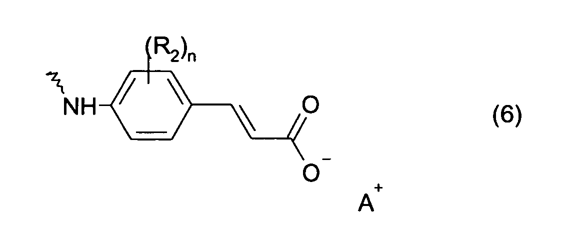 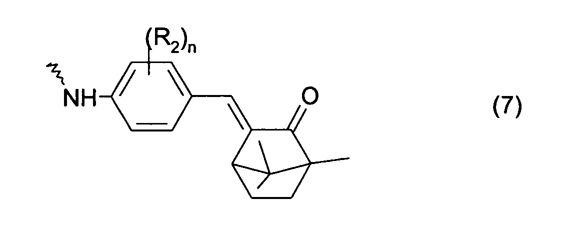 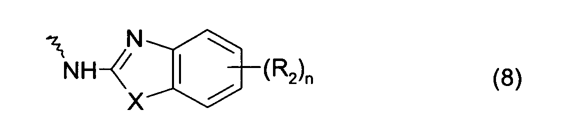
   dans lesquelles :
   R₂ représente un groupe alkyle en C₁-C₈, linéaire ou ramifié ;
   n vaut 0, 1 ou 2,
   les radicaux A, identiques ou différents représentent hydrogène ; un cation du type métal alcalin comme sodium ou potassium ; un groupe ammonium ; un radical mono, di- ou tri- alkylammonium en C₁-C₂₀ ; un radical mono, di- ou trialkanolammonium en C₂-C₂₀ ; ; un groupe héterocyclique azoté quaternaire en C₅-C₈.
   R₃ représente un radical alkyle en C₁-C₂₀, linéaire ou ramifié ; un radical alcényle en C₂-C₂₀, linéaire ou ramifié ; les dits radicaux pouvant contenir un ou plusieurs atomes d'oxygène, atomes de silicium ou groupes trisiloxanes ;
   R₄ représente un radical alkyle en C₁-C₂₀, linéaire ou ramifié ; un radical alcényle en C₂-C₂₀, linéaire ou ramifié ; un radical alcoxy en C₁-C₂₀, linéaire ou ramifié ; lesdits radicaux pouvant contenir un ou plusieurs atomes d'oxygène, atomes de silicium ou groupes trisiloxanes ;
   R₅ représente l'hydrogène ou OH,
   X représente O, S ou NR₆ avec R₆ représentant H ou un radical alkyle en C₁-C₄, linéaire ou ramifié ;
   sous réserve qu'au moins un des radicaux R₁, R₁' et R₁" désigne un groupe chromophore de formule (2) et qu'au plus un des radicaux R₁, R₁' et R₁" désigne un groupe chromophore de formule (3) ;
   (ii) OH ; un radical alcoxy en C₁-C₂₀, linéaire ou ramifié ; un radical mono ou dialkylamino en C₁-C₂₀, linéaire ou ramifié ; lesdits radicaux pouvant contenir un ou plusieurs atomes d'oxygène, atomes de silicium ou groupes trisiloxanes.

Dans la formule (I) ci-dessus, les radicaux alkyles peuvent être choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et tert.-octyle. Le radical alkyle particulièrement préféré est le radical méthyle.

Dans les formules (I) ci-dessus, les radicaux alcoxy peuvent être choisis notamment au sein des radicaux méthoxy, éthoxy, n-propoxy, isopropoxy, n-butoxy et isobutoxy. Le radical alcoxy particulièrement préféré est le radical méthoxy.

Dans la formule (I) ci-dessus, les radicaux alcènyle peuvent être choisis notamment au sein des radicaux éthylène, propylène, butène.

Dans la formule (I) ci-dessus, les radicaux mono, di ou trialkylammonium en C₁-C₂₀ peuvent être choisis parmi mono, di- ou triméthylammomnium ; ou mono, di- ou triéthylammomnium

Dans la formule (I) ci-dessus, les radicaux mono, di ou trialcanolammonium en C₂-C₂₀ peuvent être choisis parmi mono, di- ou triéthanolammomnium,

Dans la formule (I) ci-dessus, les radicaux hétérocycles quaternaires peuvent être choisi parmi pipéridinium, morpholinium, pyrrolidinium, ou pyrrolinium.

Parmi les composés de formule (I), on citera plus particulièrement ceux pour lesquels
R₁, R₁' et R₁'', identiques ou différents, désignent :
   (i) soit un chromophore de formule : 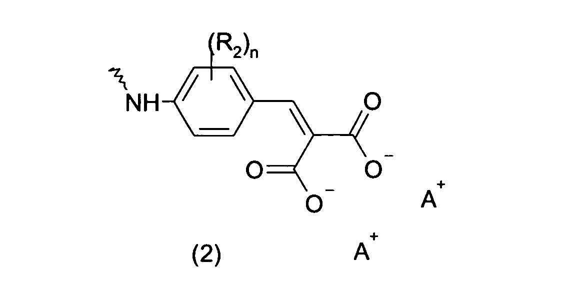 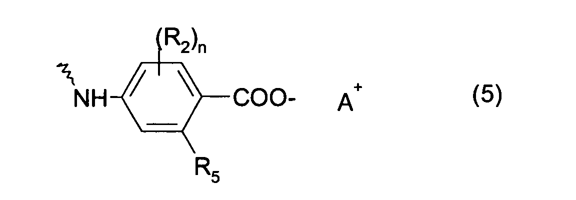 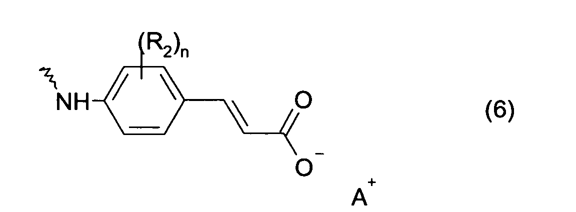 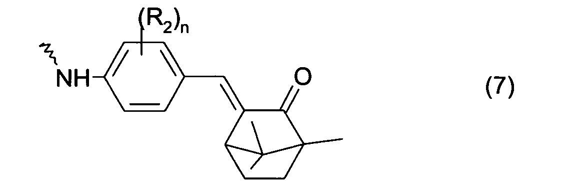 dans lesquelles :
      A désigne Na, K ou triéthanolamine ;
      n=0;
      R₅ désigne hydrogène ou OH ;
   (ii) soit un radical OH ; un radical alcoxy en C₁-C₄ et plus particulièrement méthoxy ou butoxy ; un radical dialkylamino en C₁-C₄ et plus particulièrement diméthylamino.

Parmi les composés de formule (1), on citera encore plus particulièrement ceux choisis parmi les composés suivants :

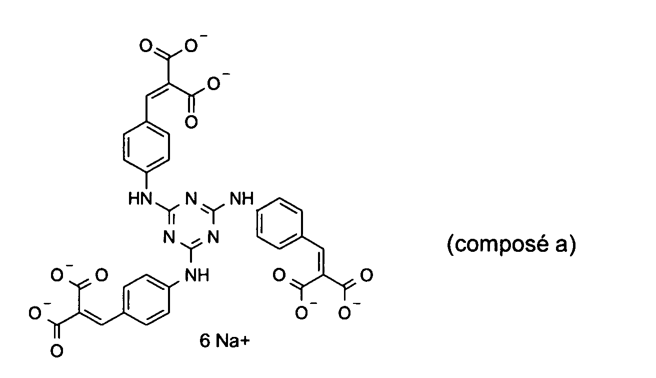

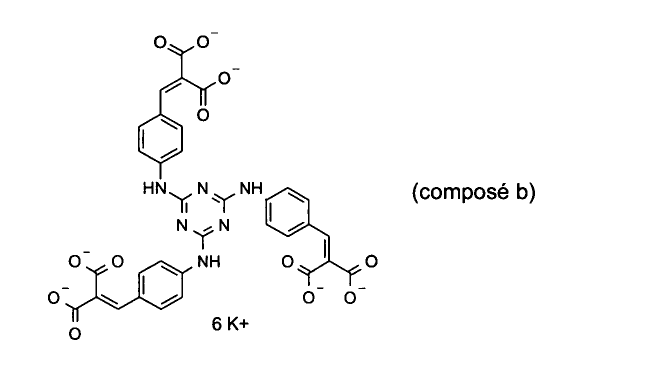

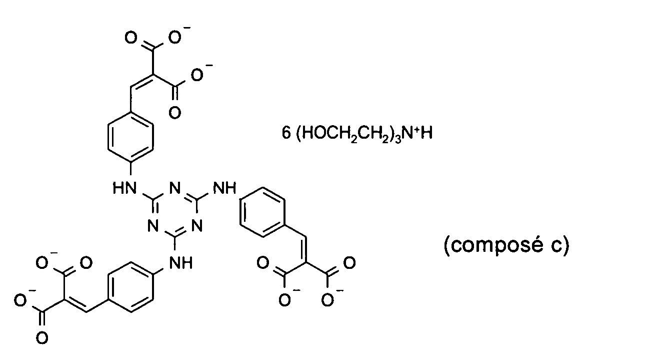

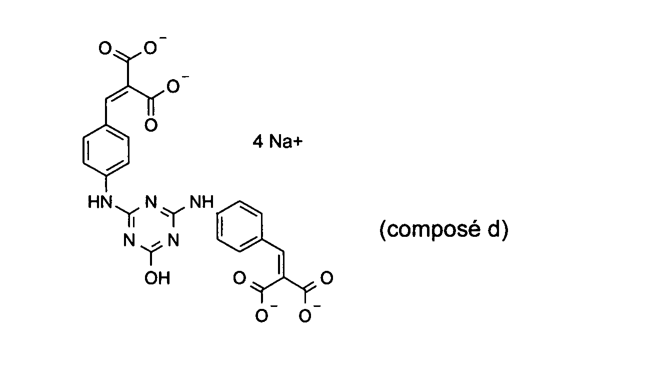

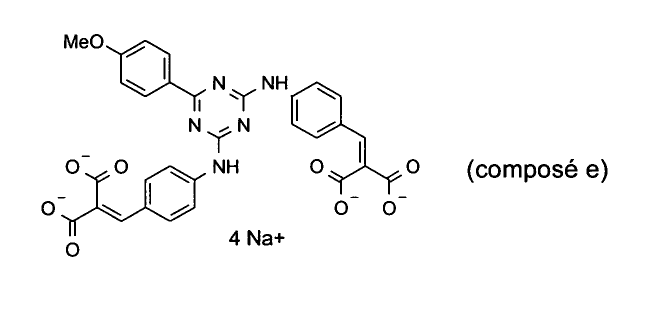

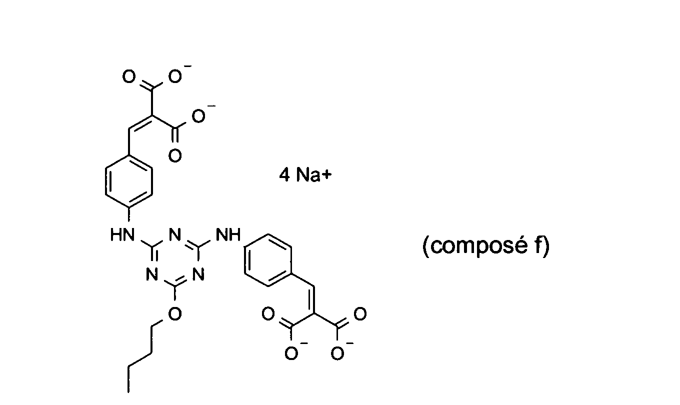

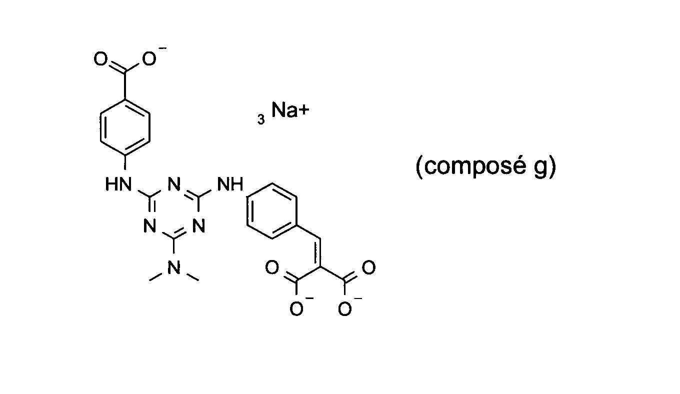

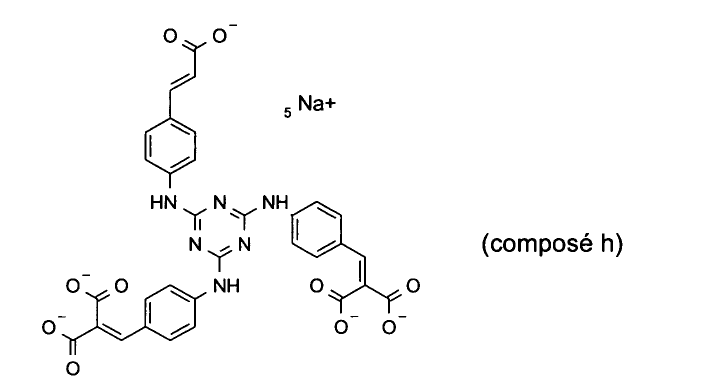

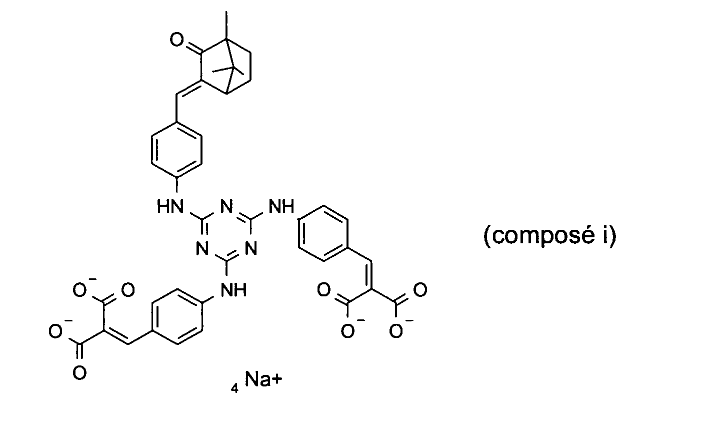

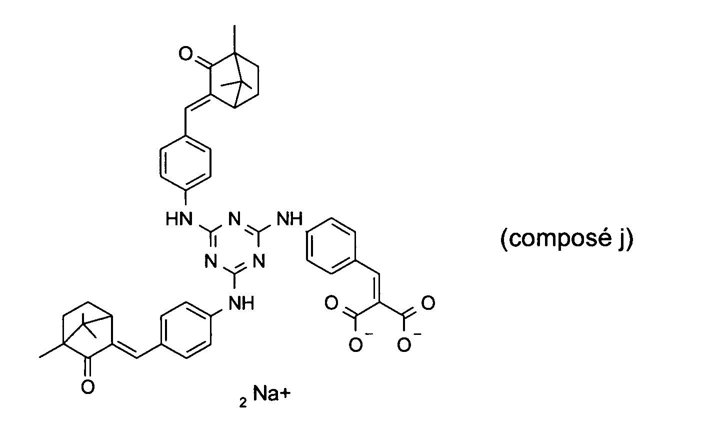

Les dérivés de formule (1) peuvent être obtenus selon le schéma suivant (Voie A) : lorsque l'un ou deux des groupements R₁, R'₁, R''₁ est ou sont différents de la formule (2), ces derniers sont d'abord greffés sur la s-triazine de la même façon que dans les brevets US 4,617,390 (groupement para amino benzoate), EP507692 (groupement para-aminobenzylidène camphre) ou EP841341 (divers groupements) en faisant réagir sur une 2,4,6-trichloro-s-triazine de formule (A) un premier composé R₁H puis un composé R'₁H selon le schéma (I) suivant 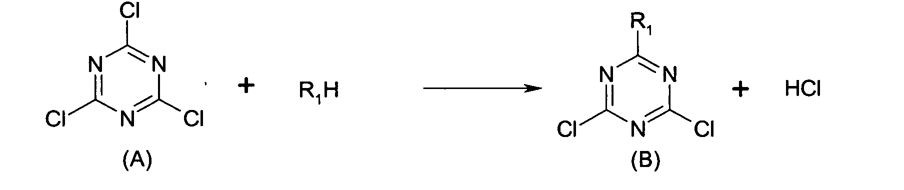 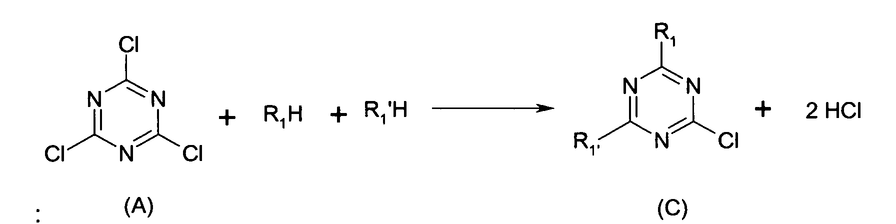 suivi du greffage d'un ou deux groupements de formule (2) sur la s-triazine (B) ou (C) ainsi obtenue par condensation d'un composé aminobenzalmalonate de formule (D) pour donner le dérivé de formule (1) selon le schéma (II) suivant : 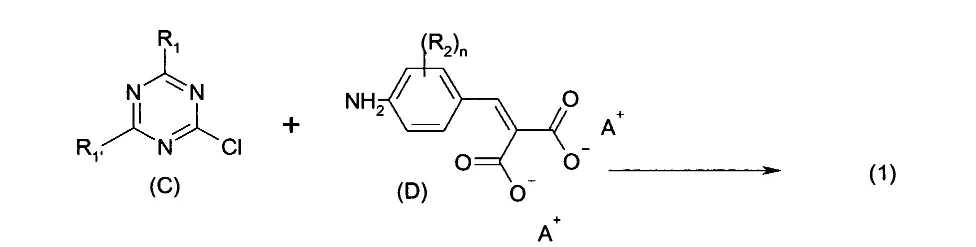 si le composé de formule (1) comporte un seul groupe de formule (2)
ou bien : 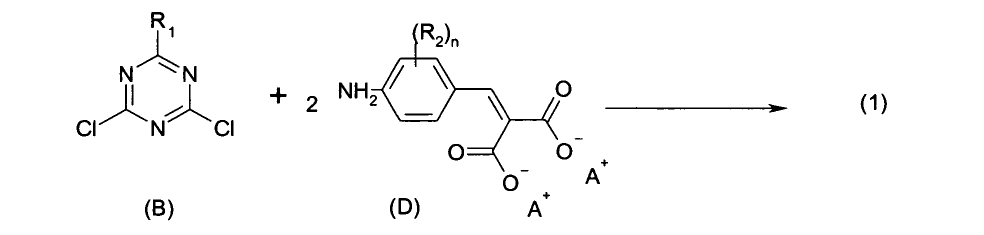 si le composé de formule (1) comporte deux groupes de formule (2)
les symboles R₁, R₁', R₂, n et A ayant les mêmes significations indiquées ci-dessus dans la formule (I).

Les composés de formule (1) comportant 3 groupes de formule (2) peuvent être obtenus selon le schéma (III) suivant :

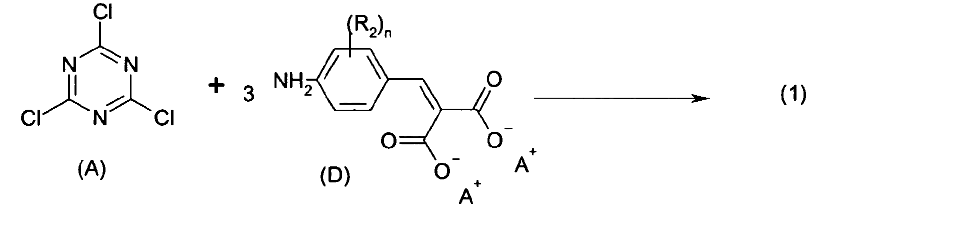

Un autre objet de la présente invention est la voie de synthèse nouvelle d'obtention des sels des dérivés d'acide benzalmalonates greffés sur s-triazine qui consiste à faire réagir sur un composé s-triazine mono-, di- ou tri-chloré de formule (A), (B) ou (C) tels que définis ci-dessus un composé aminobenzalmalonate de formule (D) tel que défini ci-dessus.

Cette synthèse s'effectue de manière générale en milieu aqueux homogène ou en suspension et en présence ou pas d'un co-solvant organique, le pH étant ajusté pendant la réaction à un pH n'excédant pas 9,5 et plus particulièrement inférieur à 9,0 et à une température comprise entre +5°C-120°C et plus particulièrement entre +5°C-85°C.
Comme co-solvant organique, on peut utiliser entre autres l'acétone, le THF ou le toluène.
Comme agent basique, on peut utiliser entre autres la soude, la potasse, le bicarbonate ou le carbonate de sodium.

Les composés de formule (1) selon l'invention peuvent être obtenus également selon une autre voie de synthèse (Voie B) qui consiste à synthétiser les esters correspondants aux composés de formule (1) et d'obtenir les composés de formule (1) par saponification de ceux ci, par exemple en présence de soude ou de potasse alcoolique avec obtention des sels sodiques et potassiques respectivement de formules (1) selon le schéma (III) suivant :

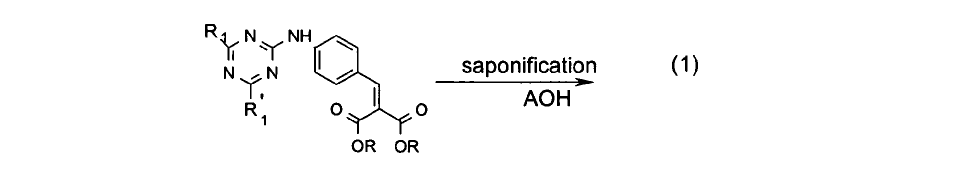

La synthèse des produits de départ à fonction ester étant par exemple décrite dans le brevet EP 0 507 691 ; pour l'obtention d'autres sels que sodique ou potassique, il faut passer par la forme acide des produits par l'addition d'acide chlorhydrique concentré jusqu'à un pH de 2, isoler la forme acide, laquelle est ensuite salifiée de manière appropriée par une amine par exemple pour l'obtention du sel d'ammonium correspondant.

Les composés de formule (I) sont généralement présents dans la composition de l'invention dans des proportions comprises entre 0,01 % et 20 % en poids, de préférence entre 0,1 % et 10 % en poids, par rapport au poids total de la composition.

Les compositions conformes à l'invention peuvent comporter en plus d'autres filtres UV organiques ou inorganiques complémentaires actifs dans l'UVA et/ou l'UVB hydrosolubles ou liposolubles ou bien insolubles dans les solvants cosmétiques couramment utilisés.

Les filtres organiques complémentaires sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrytate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US 5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres organiques complémentaires, on peut citer ceux désignés ci-dessous sous leur nom INCI :
Dérivés de l'acide para-aminobenzoique:
   PABA,
   Ethyl PABA,
   Ethyl Dihydroxypropyl PABA,
   Ethylhexyl Diméthyl PABA vendu notamment sous le nom « ESCALOL 507 » par ISP,
   Glyceryl PABA,
   PEG-25 PABA vendu sous le nom « UVINUL P25 » par BASF,
Dérivés salicyliques :
   Homosalate vendu sous le nom « Eusolex HMS » par Rona/EM Industries,
   Ethylhexyl Salicylate vendu sous le nom « NEO HELIOPAN OS » par HAARMANN et REIMER,
   Dipropyleneglycol Salicylate vendu sous le nom « DIPSAL » par SCHER,
   TEA Salicylate, vendu sous le nom « NEO HELIOPAN TS » par HAARMANN et REIMER,
Dérivés du dibenzoylméthane :
   Butyl Methoxydibenzoylmethane vendu notamment sous le nom commercial « PARSOL 1789 » par HOFFMANN LA ROCHE,
   Isopropyl Dibenzoylmethane,
Dérivés cinnamiques :
   Ethylhexyl Methoxycinnamate vendu notamment sous le nom commercial « PARSOL MCX » par HOFFMANN LA ROCHE,
   Isopropyl Methoxy cinnamate,
   Isoamyl Methoxy cinnamate vendu sous le nom commercial « NEO HELIOPAN E 1000 » par HAARMANN et REIMER,
   Cinoxate, DEA Methoxycinnamate, Diisopropyl Methylcinnamate,
   Glyceryl Ethylhexanoate Dimethoxycinnamate
Dérivés de β,β'-diphénylacrylate :
   Octocrylene vendu notamment sous le nom commercial « UVINUL N539 » par BASF,
   Etocrylene, vendu notamment sous le nom commercial « UVINUL N35 » par BASF,
Dérivés de la benzophénone :
   Benzophenone-1 vendu sous le nom commercial « UVINUL 400 » par BASF,
   Benzophenone-2 vendu sous le nom commercial « UVINUL D50 » par BASF
   Benzophenone-3 ou Oxybenzone, vendu sous le nom commercial « UVINUL M40 » par BASF,
   Benzophenone-4 vendu sous le nom commercial « UVINUL MS40 » par BASF, Benzophenone-5
   Benzophenone-6 vendu sous le nom commercial « Helisorb 11 » par Norquay
   Benzophenone-8 vendu sous le nom commercial « Spectra-Sorb UV-24 » par American Cyanamid
   Benzophenone-9 vendu sous le nom commercial «UVINUL DS-49» par BASF, Benzophenone-12
   2-(4-diethylamino-2-hydroxybenzoyl)-benzoate de n-hexyle.
Dérivés du benzylidène camphre :
   3-Benzylidene camphor fabriqué sous le nom « MEXORYL SD» par CHIMEX,
   4-Methylbenzylidene camphor vendu sous le nom « EUSOLEX 6300 » par MERCK,
   Benzylidene Camphor Sulfonic Acid fabriqué sous le nom « MEXORYL SL» par CHIMEX,
   Camphor Benzalkonium Methosulfate fabriqué sous le nom « MEXORYL SO » par CHIMEX,
   Terephthalylidene Dicamphor Sulfonic Acid fabriqué sous le nom « MEXORYL SX » par CHIMEX,
   Polyacrylamidomethyl Benzylidene Camphor fabriqué sous le nom « MEXORYL SW » par CHIMEX,
Dérivés du phenyl benzimidazole :
   Phenylbenzimidazole Sulfonic Acid vendu notamment sous le nom commercial « EUSOLEX 232 » par MERCK,
   Disodium Phenyl Dibenzimidazole Tetra-sulfonate vendu sous le nom commercial commercial « NEO HELIOPAN AP » par HAARMANN et REIMER,
Dérivés de la triazine :
   Anisotriazine vendu sous le nom commercial «TINOSORB S » par CIBA GEIGY,
   Ethylhexyl triazone vendu notamment sous le nom commercial «UVINUL T150 » par BASF,
   2,4,6-tris-(4'-amino benzalmalonate de diisobutyle)-s- triazine,
   Diethylhexyl Butamido Triazone vendu sous le nom commercial « UVASORB HEB » par SIGMA 3V,
Dérivés du phenyl benzotriazole :
   Drometrizole Trisiloxane vendu sous le nom « Silatrizole » par RHODIA CHIMIE

Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,
Dérivés anthraniliques :
   Menthyl anthranilate vendu sous le nom commercial commercial « NEO HELIOPAN MA » par HAARMANN et REIMER,
Dérivés d'imidazolines :
   Ethylhexyl Dimethoxybenzylidene Dioxoimidazoline Propionate,
Dérivés du benzalmalonate :
   Polyorganosiloxanes à fonction benzalmalonate tels que le Polysilicone-15 vendu sous la dénomination commerciale « PARSOL SLX » par HOFFMANN LA ROCHE
Dérivés de 4,4-diarylbutadiène :
   -1,1-dicarboxy (2,2'-diméthyl-propyl)-4,4-diphénylbutadiène
Dérivés de benzoxazole :
   2,4-bis-[5-1 (diméthylpropyl)benzoxazol-2-yl-(4-phenyl)-imino]-6-(2-ethylhexyl)-imino-1,3,5-triazine vendu sous le nom Uvasorb K2A par Sigma 3V ;
et leurs mélanges.

Les filtres UV organiques complémentaires préférentiels sont choisis parmi Ethylhexyl Salicylate,
Ethylhexyl Methoxycinnamate
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine et leurs mélanges.

Les filtres complémentaires inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les filtres UV complémentaires conformes à l'invention sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions cosmétiques selon l'invention peuvent contenir en outre des agents de bronzage et/ou de brunissage artificiel de la peau (agents autobronzants) tels que la dihydroxyacétone (DHA).

Les compositions conformes à la présente invention peuvent comprendre en outre des adjuvants cosmétiques classiques notamment choisis parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Les épaississants peuvent être choisis notamment parmi les polymères acryliques réticulés comme les Carbomer, les polymères réticulés acrylates/C10-C30alkylacrylates du type Pemulen ou le polyacrylate-3 vendu sous le nom VISCOPHOBE DB 1000 par Amerchol ; les polyacrylamides tels que l'émulsion polyacrylamide, C13-C14 isoparraffine et laureth-7 vendue sous le nom SEPIGEL 305 par SEPPIC, les homopolymères ou copolymères d'AMPS tel l'HOSTACERIN AMPS vendu par CLARIANT, les gommes de guar et celluloses modifiées ou non telles que la gomme de guar hydroxypropylée, la méthylhydroxyéthylcellulose et l'hydroxypropylméthyl cellulose, la gomme de xanthane, les silices nanométriques de type Aerosil.

Bien entendu, l'homme de l'art veillera à choisir le ou les éventuels composés complémentaires cités ci-dessus et/ou leurs quantités de manière telle que les propriétés avantageuses attachées intrinsèquement aux composés conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, d'une huile, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Lorsque la composition cosmétique selon l'invention est utilisée pour le soin de l'épiderme humain, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, d'huile solaire, de bâtonnet solide, de poudre, de mousse aérosol ou de spray.

Lorsque la composition cosmétique selon l'invention est utilisée pour le soin des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des ongles, des lèvres, des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Un autre objet de l'invention est l'utilisation d'un composé de formule (I) tel que définie ci-dessus dans une composition cosmétique ou dermatologique comme agent filtrant les radiations UV.

Un autre objet de l'invention est l'utilisation d'un composé de formule (I) tel que définie ci-dessus dans une composition cosmétique comme agent de contrôle de la variation de la couleur de la peau due aux rayonnements UV.

Un autre objet de l'invention est l'utilisation d'un composé de formule (I) tel que définie précédemment comme agent photostabilisant de polymères synthétiques tels que les matières plastiques ou de verres en particulier des verres de lunettes ou des lentilles de contact.

Des exemples concrets, mais nullement limitatifs, illustrant l'invention, vont maintenant être donnés.

### EXEMPLE 1 : Préparation du sel hexasodique de l'acide 2-(4-{4,6-Bis-[4-(2,2-dicarboxy-vinyl)-phénylamino]-[1,3,5]triazin-2-ylamino}-benzylidène)-malonique par la voie A :

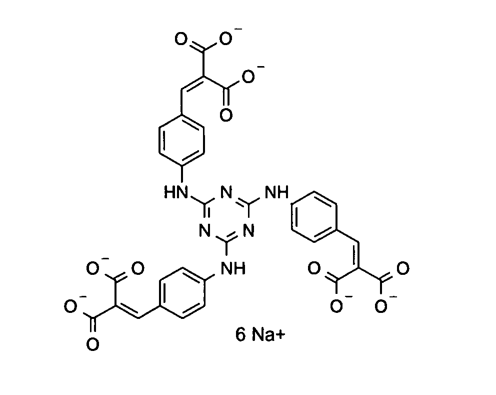

### Première étape : préparation du sel di-sodique de l'acide 2-(4-aminobenzylidène)-malonique :

Dans un réacteur équipé d'un thermomètre, d'une agitation mécanique et d'un réfrigérant, on place la pipéridine (206,6g, 2,42 mole). On chauffe vers 40°C. On introduit l'acide malonique par portions en 15 minutes sans dépasser 70°C (83,4g, 0,8 mole). On maintient la pâte visqueuse obtenue à 70°C et introduit par portions le p-amino benzaldéhyde en 15 minutes (97g, 0,8 mole). Le mélange visqueux est laissé à 90°C sous agitation et contrôle de viscosité. Lorsque la viscosité devient plus importante, on commence à introduire l'alcool isopropylique (après 1 heure 15 minutes). L'introduction de 200 ml d'alcool isopropylique s'est faite en 2 heures 50 minutes. On refroidit et on filtre. Le précipité obtenu est lavé avec un minimum d'alcool isopropylique. Après séchage sous vide sur P₂O₅, on obtient 210 g (Rendement 70 %) du sel de di-pépiridinium de l'acide 2-(4-amino-benzylidène)-malonique sous forme de poudre jaune. Ensuite, on introduit de la soude (200ml de soude aqueuse à 35%, 1,75 mole) en 15 minutes à une température de 90°C-70°C toujours sous agitation. On refroidit vers 40°C. On ajoute 700 ml d'éthanol et filtre le mélange réactionnel. Le solide est repris dans au moins 500 ml d'eau et filtré. On obtient environ 5 g de résidu que l'on jette. Le filtrat est concentré de moitié et on ajoute 700 ml d'éthanol à 95 et laisse cristalliser. Après filtration, séchage sous vide sur P₂O₅, on obtient 140 g (Rendement 70 %) du sel di-sodique de l'acide 2-(4-amino-benzylidène)-malonique sous forme de poudre jaune utilisée telle quelle dans l'étape suivante.

### Deuxième étape : préparation de l'acide 2-(4-{4,6-Bis-[4-(2,2-dicarboxy-vinyl)-phénylamino]-[1,3,5]triazin-2-ylamino}-benzylidène)-malonique :

Dans un réacteur équipé d'un thermomètre, d'un système de mesure de pH, d'une agitation mécanique, d'un réfrigérant et d'une ampoule d'introduction, le dérivé obtenu à l'étape précédente (108g, 0,43 mole) est solubilisé dans 300 ml d'eau et est maintenu à +5°C. On ajoute à température inférieure à +5°C le chlorure de cyanuryle (18,4g, 0,3 équivalent chlore) dissous dans 350 ml d'acétone. En fin d'introduction, le pH doit être égal à 7 environ. Le mélange hétérogène est chauffé progressivement, l'acétone est distillée pour pouvoir chauffer le mélange réactionnel à 95°C. Le pH est ajusté et maintenu à pH 9 avec de la soude concentrée. On chauffe pendant 2 heures. Après refroidissement, les jus sont acidifiés jusqu' à pH 1 par de l'acide chlorhydrique concentré. Il se forme un fin précipité orangé. On le filtre. Le gâteau obtenu est redispersé dans l'eau et filtré. Après filtration, séchage sous vide sur P₂O₅, on obtient 56,6 g de poudre orangée (Rendement = 81 %) du dérivé acide de l'exemple 1 :

| | | | |
|---|---|---|---|
| UV (Ethanol) | λₘₐₓ = 348 nm | εₘₐₓ = 64 820 | E_{1%} = 930 |

### Troisième étape : préparation du dérivé de l'exemple 1 :

Le dérivé de l'étape précédente (5g, 0,0072 mole) est suspendu dans 19,9 ml d'eau. On y ajoute sous agitation de la soude à 35% dans l'eau (4,9g, 0,043 mole). On obtient ainsi le dérivé sel de sodium de l'exemple 1 sous forme d'une solution aqueuse à 20% de matière active (sel de sodium) et de couleur brune (pH 9,8) :

| | | | |
|---|---|---|---|
| UV (Eau) | λₘₐₓ = 323 nm | εₘₐₓ = 86 428 | E_{1%} = 1043. |

### EXEMPLE 2 : Préparation du sel hexasodique de l'acide 2-(4-{4,6-Bis-[4-(2,2-dicarboxy-vinyl)-phénylamino]-[1,3,5]triazin-2-ylamino}-benzylidène)-malonique par la voie B :

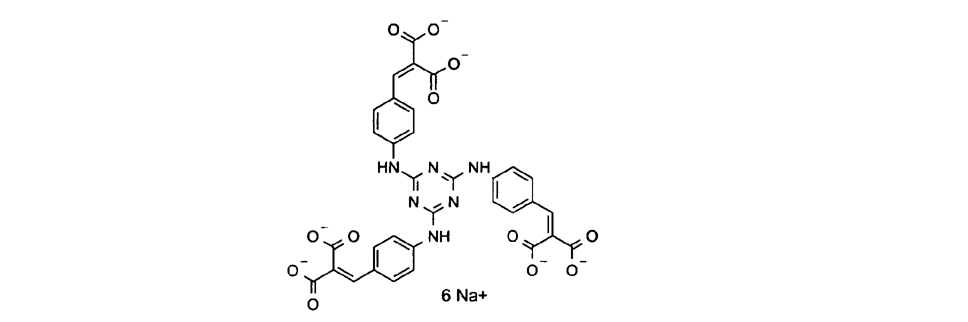

### Première étape : préparation de l'acide 2-(4-{4,6-Bis-[4-(2,2-dicarboxy-vinyl)-phénylamino]-[1,3,5]triazin-2-ylamino}-benzylidène)-malonique :

Le 2,4,6-tris(4'-amino benzalmalonate de diisobutyle)-s-triazine (exemple 1 du brevet EP 507 691 B1) (10,3g, 0,01 mole) est solubilisé dans 150ml d'isopropanol. La potasse en excès (7,8g, 0,12 mole) dissoute dans 50ml d'isopropanol y est ajoutée. On porte au reflux pendant 2 heures. Un insoluble se forme. On filtre et lave le précipité obtenu à l'isopropanol. Le solide est mis en solution dans 50ml d'eau. On ajuste le pH à 1 avec de l'acide chlorhydrique concentré sous agitation. Le solide obtenu est filtré, lavé à l'eau puis séché. On obtient 5,2g (Rendement 67%) du dérivé acide de l'exemple 2 sous forme d'une poudre jaune.

### Deuxième étape : préparation du dérivé de l'exemple 2 :

Le dérivé de l'étape précédente (5g, 0,0072 mole) est suspendu dans 19,9 ml d'eau. On y ajoute sous agitation de la soude à 35% dans l'eau (4,9g, 0,043 mole). On obtient ainsi le dérivé sel de sodium de l'exemple 1 sous forme d'une solution aqueuse à 20% de matière active (sel de sodium) et de couleur brun pâle (pH 9,6) :

| | | | |
|---|---|---|---|
| UV (Eau) | λₘₐₓ = 323 nm | εₘₐₓ = 87 820 | E_{1%} = 1060. |

### EXEMPLE 3 : Préparation du sel hexapotassique de l'acide 2-(4-{4,6-Bis-[4-(2,2-dicarboxy-vinyl)-phénylamino]-[1,3,5]triazin-2-ylamino}-benzylidène)-malonique par la voie B :

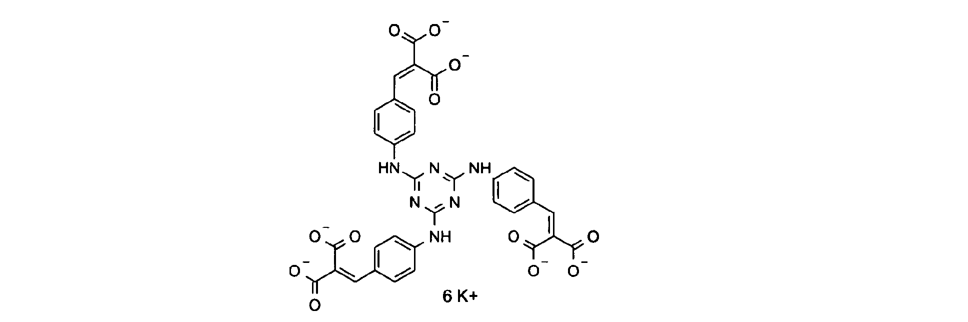

Le 2,4,6-tris(4'-amino benzalmalonate de diisobutyle)-s-triazine (exemple 1 du brevet EP 507 691 B1) (10,3g, 0,01 mole) est solubilisé dans 150ml d'isopropanol. La potasse en excès (7,8g, 0,12 mole) dissoute dans 50ml d'isopropanol y est ajoutée. On porte au reflux pendant 2 heures. Un insoluble se forme. On filtre et lave le précipité obtenu copieusement à l'isopropanol. Après séchage sous vide sur P₂O₅, on obtient 9,2 g (Rendement = 100 %) du dérivé de l'exemple 3 sous forme d'un solide rouge brique qui devient jaune à l'air :

| | | | |
|---|---|---|---|
| UV (Eau) | λₘₐₓ = 324 nm | εₘₐₓ = 66 600 | E_{1%} = 720 |

### EXEMPLE 4 : Préparation du sel hexatriéthanolamine de l'acide 2-(4-{4,6-Bis-[4-(2,2-dicarboxy-vinyl)-phénylamino]-[1,3,5]triazin-2-ylamino}-benzylidène)-malonique par la voie A :

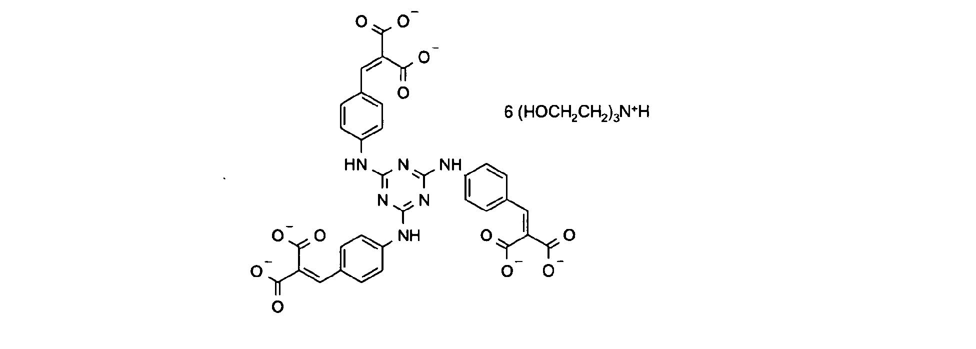

Le dérivé de la deuxième étape de l'exemple 1 (5g, 0,0072 mole) est suspendu dans 26,6 ml d'eau. On y ajoute sous agitation de la triéthanolamine (6,4g, 0,043 mole). On obtient ainsi le dérivé sel de triéthanolamine de l'exemple 4 sous forme d'une solution aqueuse à 30% de matière active (sel de triéthanolamine) et de couleur brun pâle (pH 7,3) :

| | | | |
|---|---|---|---|
| UV (Eau) | λₘₐₓ = 322 nm | εₘₐₓ = 83 250 | E_{1%} = 523. |

### EXEMPLE 5 :

### Préparation du sel tetrasodique de l'acide 2-(4-{4-[4-(2,2-dicarboxy-vinyl)-phénylamino]-6-hydroxy-[1,3,5]triazin-2-ylamino}-benzylidène)-malonique par la voie A :

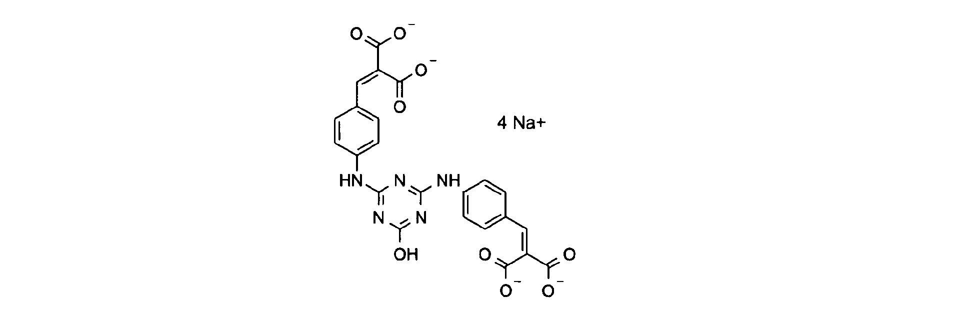

### Première étape : préparation du sel di-potassique de l'acide 2-(4-amino-benzylidène)-malonique :

Le sel de di-pépiridinium de l'acide 2-(4-amino-benzylidène)-malonique obtenu à la première étape de l'exemple 1 (210g) est ajouté à de la potasse à 85% (114g, 1,75 mole) dissoute dans 200ml d'éthanol à 96% en 15 minutes à une température de 90°C-70°C toujours sous agitation.

On refroidit vers 40°C. On ajoute 700 ml d'éthanol à 96% et filtre le mélange réactionnel. Le solide est repris dans au moins 500 ml d'eau et filtré. Le filtrat est concentré de moitié et on ajoute 500 ml d'isopropanol et laisse cristalliser.

Après filtration, séchage sous vide sur P₂O₅, on obtient 205g (Rendement 90 %) du sel di-potassique de l'acide 2-(4-amino-benzylidène)-malonique sous forme d'une poudre jaune clair utilisée telle quelle dans l'étape suivante.

### Deuxième étape : préparation de l'acide 2-(4-{4-chloro-6-[4-(2,2-dicarboxyvinyl)-phénylamino]-[1,3,5]triazin-2-ylamino}-benzylidène)-malonique :

Dans un réacteur équipé d'un thermomètre, d'un système de mesure de pH, d'une agitation mécanique, d'un réfrigérant et d'une ampoule d'introduction, le dérivé obtenu à l'étape précédente (124,6g, 0,44 mole) est solubilisé dans 800 ml d'eau et est maintenu à +5°C. On ajoute à température inférieure à +5°C le chlorure de cyanuryle (36,9g, 0,2 équivalent chlore) dissous dans 700 ml d'acétone. En fin d'introduction, le pH doit être égal à 7 environ. Le mélange hétérogène est chauffé progressivement, l'acétone est distillée pour pouvoir chauffer le mélange réactionnel à 95°C. Le pH est ajusté et maintenu à pH 9 avec de la potasse. On chauffe pendant 2 heures. Après refroidissement, les jus sont acidifiés jusqu' à pH 1 par de l'acide chlorhydrique concentré. Il se forme un fin précipité jaune orangé. On le filtre. Le gâteau obtenu est redispersé dans l'eau et filtré. Après filtration, séchage sous vide sur P₂O₅, on obtient 91,3 g (Rendement = 90 %) de l'acide 2-(4-{4-chloro-6-[4-(2,2-dicarboxy-vinyl)-phénylamino]-[1,3,5]triazin-2-ylamino}-benzylidène)-malonique sous forme de poudre rouge orangée:

| | | | |
|---|---|---|---|
| UV (NaOH 0,1N) | λₘₐₓ=320nm | εₘₐₓ = 64 960 | E_{1%} = 932 |

### Troisième étape : préparation de l'acide 2-(4-{4-[4-(2,2-dicarboxy-vinyl)-phénylamino]-6-hydroxy-[1,3,5]triazin-2-ylamino}-benzylidène)-malonique:

Le dérivé de l'étape précédente (50g, 0,095 mole) est mis en suspension dans 500 ml d'eau. On y ajoute sous agitation de la soude à 35% dans l'eau jusqu'à l'obtention d'un pH de 12. On chauffe à 95°C sous agitation pendant 2 heures. On refroidit. Le milieu est acidifié jusqu'à un pH de 1 par de l'acide chlorhydrique concentré. Après filtration, copieux lavages à l'eau et séchage sous vide sur P₂O₅, on obtient 52,4g (Rendement = 100%) de l'acide 2-(4-{4-[4-(2,2-dicarboxy-vinyl)-phénylamino]-6-hydroxy-[1,3,5]triazin-2-ylamino}-benzylidène)-malonique sous forme de poudre rouge orangée:

| | | | |
|---|---|---|---|
| UV (dmso/Ethanol) | λₘₐₓ = 336 nm | εₘₐₓ = 35 070 | E_{1%} = 691 |

### Quatrième étape : préparation du dérivé de l'exemple 5 :

Le dérivé obtenu à l'étape précédente (10g, 0,02 mole) est suspendu dans 43,8 ml d'eau. On y ajoute sous agitation de la soude à 35% (3,16g, 0,079 mole). On obtient ainsi le dérivé sel de sodium de l'exemple 5 sous forme d'une solution aqueuse à 20% de matière active (sel de sodium) et de couleur brun pâle (pH 9,2) :

| | | | |
|---|---|---|---|
| UV (NaOH 0,1N) | λₘₐₓ = 322 nm | εₘₐₓ = 50 700 | E_{1%} = 852. |

### EXEMPLE 6 : Préparation du sel tetrasodique de l'acide 2-{4-[4-[4-(2,2-dicarboxy-vinyl)-phénylamino]-6-(4-méthoxy-phényl)-[1,3,5]triazin-2-ylamino]-benzylidène}-malonique par la voie A :

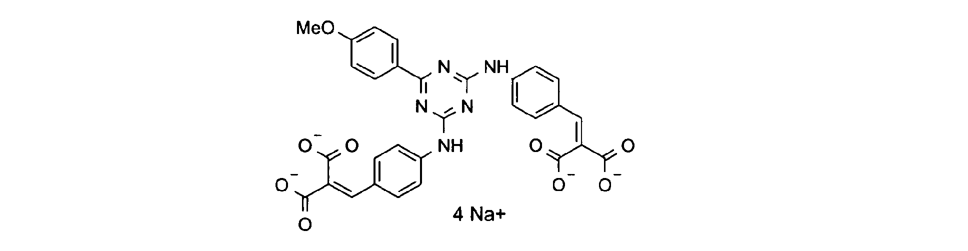

### Première étape : préparation du 2,4-dichloro-6-(4-méthoxy-phényl)-[1,3,5]triazine

Dans un réacteur de 1 litre sous azote, on charge le chlorure de cyanuryle (36,9g, 0,2 mole), puis 50ml de THF. La suspension est refroidie à 5-10°C. Sous agitation, à une température inférieure à 10°C, on coule la solution 0,5M dans le THF de para méthoxy phényl magnésium (400ml, 0,2 mole) sous gaz inerte en 45 minutes. Le mélange réactionnel est laissé une nuit au repos. Le solvant est évaporé au rotavapeur. Le résidu est versé dans l'eau acidulée. Après une extraction au dichlorométhane, on lave.la phase organique à l'eau, la sèche sur sulfate de sodium. On évapore le solvant. Le solide brut obtenu est trituré avec de l'isopropanol puis filtré. Le solide est solubilisé dans le toluène à chaud. Un ajout du même volume d'heptane précipite le produit. On refroidit, filtre et sèche. On obtient ainsi 31,7g (Rendement 62%) de 2,4-dichloro-6-(4-méthoxyphényl)-[1,3,5]triazine sous forme d'une poudre beige (Pf 133-136°C) utilisée telle quelle dans l'étape suivante.

### Deuxième étape : préparation de l'acide 2-{4-[4-[4-(2,2-dicarboxy-vinyl)-phenylamino]-6-(4-methoxy-phenyl)-[1,3,5]triazin-2-ylamino]-benzylidene}-malonique

Le sel di-sodique de l'acide 2-(4-amino-benzylidène)-malonique (obtenu à la première étape de l'exemple 1) (10,4g, 0,04 mole) est solubilisé dans 50ml d'eau. On ajoute le dérivé de l'étape précédente (5,1g, 0,02 mole) en solution dans 100ml d'acétone à 40°C. On maintient le pH à 12 par l'introduction de soude concentrée. On distille l'acétone jusqu'à la température de 75°C. Le milieu devient pratiquement homogène. Au bout d'une heure, la réaction est arrêtée et refroidie. On ajuste le pH à 1 avec de l'acide chlorhydrique concentré sous agitation. Le solide obtenu est filtré, lavé à l'eau puis séché. On obtient 9,7g (Rendement 81%) du dérivé acide de l'exemple 6 sous forme d'un solide rouge orangé :

| | | | |
|---|---|---|---|
| UV (Ethanol) | | | |
| λₘₐₓ = 337 nm | εₘₐₓ = 47 200 | E_{1%} = 790 | |
| λₘₐₓ = 306 nm(épaulement) | | εₘₐₓ = 37 900 | E_{1%} = 634 |

### Troisième étape : préparation du dérivé de l'exemple 6

Le dérivé de l'étape précédente (5g, 0,0084 mole) est suspendu dans 22 ml d'eau. On y ajoute sous agitation de la soude à 35% dans l'eau (1,35g, 0,0334 mole). On obtient ainsi le dérivé sel de sodium de l'exemple 6 sous forme d'une solution aqueuse à 20% de matière active et de couleur brune (pH 9,5) :

| | | | |
|---|---|---|---|
| UV (Eau) | λₘₐₓ = 318 nm | εₘₐₓ = 45 900 | E_{1%} = 768. |

### EXEMPLE 7 : Préparation du sel tetrasodique de l'acide 2-(4-{4-butoxy-6-[4-(2,2-dicarboxy-vinyl)-phénylamino]-[1,3,5]triazin-2-ylamino}-benzylidène)-malonique par la voie A :

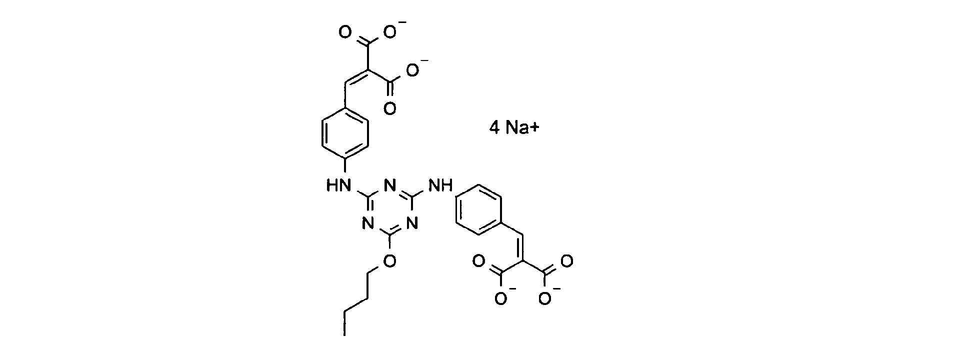

### Première étape : préparation de l'acide 2-(4-{4-butoxy-6-[4-(2,2-dicarboxy-vinyl)-phénylamino]-[1,3,5]triazin-2-ylamino}-benzylidène)-malonique :

Le sel di-sodique de l'acide 2-(4-amino-benzylidène)-malonique (obtenu à la première étape de l'exemple 1) (11,31g, 0,045 mole) est solubilisé dans 50ml d'eau. On ajoute du 2-n-butoxy-4,6-dichloro-1,3,5-triazine (5g, 0,0225 mole) en solution dans 60ml d'acétone à 50°C en 30 minutes. On maintient le pH à 9 par l'introduction de soude. On distille l'acétone jusqu'à la température de 75°C. Le milieu reste homogène. Au bout de 2 heures, la réaction est arrêtée et refroidie. On ajuste le pH à 1 avec de l'acide chlorhydrique sous agitation. Le solide obtenu est filtré, lavé à l'eau puis séché. On obtient 7,15g (Rendement 56%) du dérivé acide de l'exemple 7 sous forme d'une poudre rouge orangé très fine.

### Deuxième étape : préparation du dérivé de l'exemple 7

Le dérivé de l'étape précédente (5g, 0,0089 mole) est suspendu dans 22,5 ml d'eau. On y ajoute sous agitation de la soude à 35% dans l'eau (1,42g, 0,0335 mole). On obtient ainsi le dérivé sel de sodium de l'exemple 7 sous forme d'une solution aqueuse à 20% de matière active et de couleur brune (pH 9,2) :

| | | | |
|---|---|---|---|
| UV (Eau) | λₘₐₓ = 317 nm | εₘₐₓ = 51 960 | E_{1%} = 922. |

### EXEMPLE 8 : Préparation du sel trisodique de l'acide 2-{4-[4-carboxy-phénylamino)-6-diméthylamino-[1,3,5]triazin-2-ylamino]-benzylidène}-malonique par la voie A :

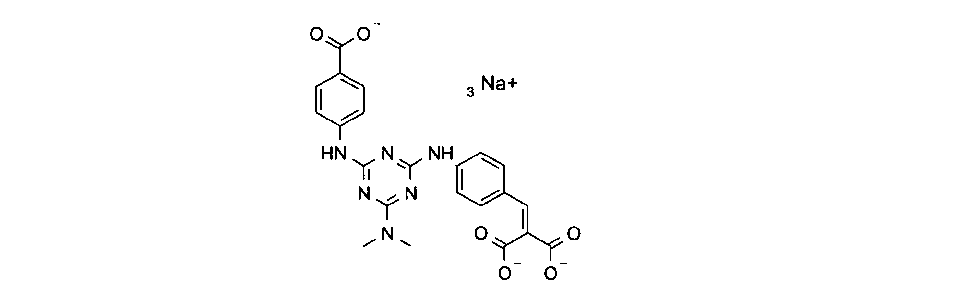

### Première étape : préparation de la 2-(4'-ylamino benzoate de butyle)-4,6-dichloro-s-triazine :

A une solution de chlorure de cyanuryle (18,4 g, 0,1 mole) dans 100 ml d'acétone/50 ml d'eau, on ajoute à 0-5°C, goutte à goutte, le 4-amino benzoate de butyle (19,32 g, 0,1 mole) en solution dans 200 ml d'acétone, puis 100 ml de bicarbonate de sodium 0,1 N en 1 heure. L'agitation est maintenue 1 heure. Après essorage du précipité, lavage à l'eau et séchage, on obtient la 2-(4'-ylamino benzoate de butyle)-4,6-dichloro-s-triazine (33 g, rendement = 96 %) sous forme d'une poudre blanche (Pf 248°C) utilisé telle quelle dans l'étape suivante.

### Deuxième étape : préparation de l'acide 2-{4-[4-carboxy-phénylamino)-6-diméthylamino-[1,3,5]triazin-2-ylamino]-benzylidène}-malonique :

Le sel di-sodique de l'acide 2-(4-amino-benzylidène)-malonique (obtenu à la première étape de l'exemple 1) (2,94g, 0,012 mole, 100% excès) est solubilisé dans 20ml d'eau. On y ajoute le dérivé précédent (2g, 0,0058 mole) en solution dans 30ml d'acétone à 50°C en 30 minutes en présence de 5ml de DMF pour homogénéiser le milieu (ce DMF a dû libérer de la N,N-diméthylamine). On maintient le pH à 12 par l'introduction de soude concentrée. On distille l'acétone jusqu'à la température de 70°C. Le milieu reste homogène. Au bout de 2 heures, la réaction est arrêtée et refroidie. On ajuste le pH à 1 avec de l'acide chlorhydrique concentré sous agitation. Le solide obtenu est filtré, lavé à l'eau puis séché. On obtient 2,34g (Rendement 85%) du dérivé acide de l'exemple 8 sous forme d'une poudre jaune :

| | | | |
|---|---|---|---|
| UV (DMF) | λₘₐₓ = 338 nm | εₘₐₓ = 34 440 | E_{1%} = 740 |
| UV (DMF) | λₘₐₓ = 303 nm | εₘₐₓ = 40 370 | E_{1%} = 870 |

### Troisième étape : préparation du dérivé de l'exemple 8

Le dérivé de l'étape précédente (1g, 0,0022 mole) est suspendu dans 4,57 ml d'eau. On y ajoute sous agitation de la soude à 35% dans l'eau (0,264g, 0,0066 mole). On obtient ainsi le dérivé sel de sodium de l'exemple 8 sous forme d'une solution aqueuse à 20% de matière active et de couleur jaune (pH 9,5) :

| | | | |
|---|---|---|---|
| UV (Eau) | λₘₐₓ = 317 nm(épaulement) | εₘₐₓ = 43 750 | E_{1%} = 943 |
| | λₘₐₓ = 302 nm | εₘₐₓ = 46 630 | E_{1%} = 1005. |

### EXEMPLE 9 : Préparation du sel pentasodique de l'acide 2-(4-{4-[4-(2-carboxy-vinyl)-phénylamino]-6-[4-(2,2-dicarboxy-vinyl)-phénylamino]-[1,3,5]triazin-2-ylamino}-benzylidène]-malonique par la voie B :

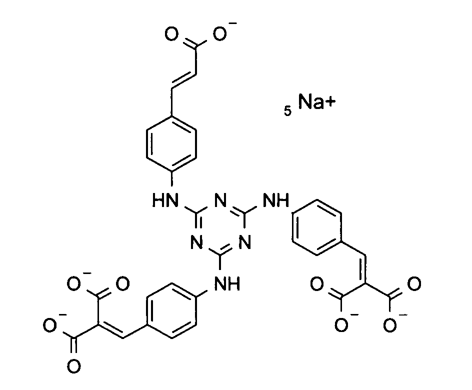

### Première étape : préparation de l'acide 2-{4-[4-carboxy-phénylamino)-6-diméthylamino-[1,3,5]triazin-2-ylamino]-benzylidène}-malonique selon la voie B :

Le 2,4-bis(4'-aminobenzalmalonate de diisobutyle)-6-(2-éthylhexylamino)-s-triazine (obtenu à l'exemple 6 du brevet EP 0 507 691) (3g, 0,003 mole) est solubilisé dans 40ml d'isopropanol. La potasse (2,6g, 0,058 mole) dissoute dans 20ml d'isopropanol y est ajoutée. On porte au reflux. Un insoluble se forme. On ajoute progressivement de l'eau et continue le chauffage pendant 2 heures. On évapore l'isopropanol. On ajuste le pH à 1 avec de l'acide chlorhydrique concentré sous agitation. Le solide obtenu est filtré, lavé à l'eau puis séché. On obtient 1,32g (Rendement 67%) du dérivé acide de l'exemple 9 sous forme d'une poudre jaune :

### Deuxième étape : préparation du dérivé de l'exemple 9 :

Le dérivé de l'étape précédente (1g, 0,00153 mole) est suspendu dans 4,53 ml d'eau. On y ajoute sous agitation de la soude à 35% dans l'eau (0,31g, 0,077 mole). On obtient ainsi le dérivé sel de sodium de l'exemple 9 sous forme d'une solution aqueuse à 20% de matière active et de couleur jaune (pH 9,5) :

| | | | |
|---|---|---|---|
| UV (Eau) | λₘₐₓ = 327 nm | εₘₐₓ = 83 070 | E_{1%} = 1274. |

### EXEMPLE 10 : Préparation du sel tetrasodique de l'acide 2-(4-{4-[4-(2-dicarboxy-vinyl)-phénylamino]-6-[4-(4,7,7-triméthyl-3-oxo-bicyclo[2.2.1]hept-2-ylidèneméthyl)-phénylamino]-[1,3,5]triazin-2-ylamino}-benzylidène]-malonique par la voie B:

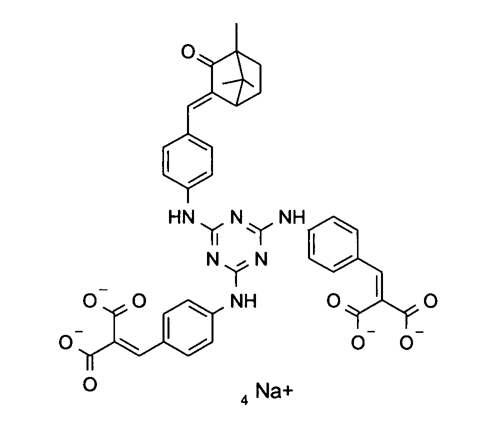

### Première étape : préparation de de l'acide 2-(4-{4-[4-(2-dicarboxy-vinyl)-phénylamino]-6-[4-(4,7,7-triméthyl-3-oxo-bicyclo[2.2.1]hept-2-ylidèneméthyl)-phénylamino]-[1,3,5]triazin-2-ylamino}-benzylidène]-malonique :

Le 2,4-bis(4'-aminobenzalmalonate de diisobutyle)-6-(4'-aminobenzylidène camphre)-s-triazine (obtenu à l'exemple 9 du brevet EP 0 507 691) (7g, 0,0072 mole) est solubilisé dans 100ml d'isopropanol. La potasse (3,24g, 0,058 mole) dissoute dans 20ml d'isopropanol y est ajoutée. On porte au reflux pendant 1 heure. L' insoluble formé est filtré et est lavé copieusement à l'isopropanol. Le solide rouge brique obtenu devient jaune à l'air. On reprend le solide dans l'eau et on acidifie jusqu'à pH 1 par de l'acide chlorhydrique. Le solide obtenu est filtré, lavé à l'eau puis séché. On obtient 4,6g (Rendement 86%) du dérivé acide de l'exemple 10 sous forme d'une poudre jaune orangée :

### Deuxième étape : préparation du dérivé de l'exemple 10

Le dérivé de l'étape précédente (2g, 0,00268 mole) est suspendu dans 8,73 ml d'eau. On y ajoute sous agitation de la soude à 35% dans l'eau (0,43g, 0,0107 mole). On obtient ainsi le dérivé sel de sodium de l'exemple 10 sous forme d'une solution aqueuse à 20% de matière active et de couleur jaune pâle (pH 9,0) :

| | | | |
|---|---|---|---|
| UV (Eau) | λₘₐₓ= 329 nm | εₘₐₓ = 69 210 | E_{1%} = 963. |

### EXEMPLE 11 : Préparation du sel disodique de l'acide 2-(4-{[4,6-bis({4-(4,7,7-triméthyl-3-oxobicyclo[2.2.1]hept-2-ylidène)méthyl]-phényl}amino)-[1,3,5]triazin-2-ylamino}-benzylidène]-malonique par la voie B:

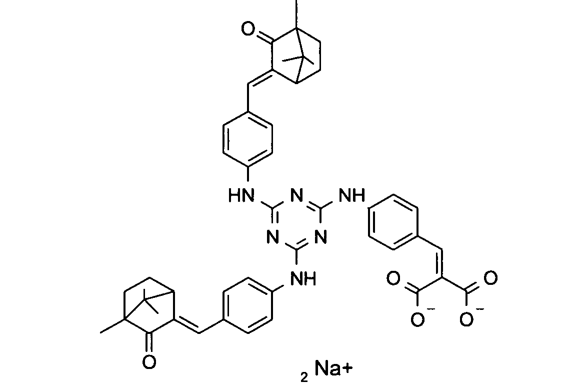

### Première étape : préparation de de l'acide 2-(4-{[4,6-bis({4-(4,7,7-triméthyl-3-oxobicyclo[2.2.1]hept-2-ylidène)méthyl]-phényl}amino)-[1,3,5]triazin-2-ylamino}-benzylidène]-malonique :

Le 2,4-bis(4'-aminobenzylidène camphre)-6-(4'-aminobenzalmalonate de diisobutyle)--s-triazine (obtenu à l'exemple 2 du brevet EP 0 507 692) (1,5g, 0,0017 mole) est solubilisé dans un mélange d'éthanol et de soude aqueuse. On porte au reflux pendant 7 heures. L' insoluble formé est filtré et est lavé copieusement à l'isopropanol. Après refroidissement, un précipité se forme. On filtre. Les jus de filtration sont acidifiés jusqu'à pH 1 par de l'acide chlorhydrique. Le solide obtenu est filtré, lavé à l'eau puis séché. On obtient 0,33g (Rendement 24%) du dérivé acide de l'exemple 11 sous forme d'une poudre jaune

### Deuxième étape : préparation du dérivé de l'exemple 11

Le dérivé de l'étape précédente (0,33g, 4,2x10-4 mole) est suspendu dans 7,03 ml d'eau. On y ajoute sous agitation de la soude à 35% dans l'eau (0,033g, 4,2x10-4 mole). On obtient ainsi le dérivé sel de sodium de l'exemple 11 sous forme d'une solution aqueuse à 5% de matière active et de couleur jaune pâle (pH 8,5) : UV (Eau) λₘₐₓ = 353 nm εₘₐₓ = 98 170 E_{1%} = 1238.

### EXEMPLE 12 : Composition antisolaire (émulsion huile-dans-eau)

- Composé de l'exemple 4 (solution aqueuse sel de triéthanolamine à 30% MA) 6 g
- Mélange d'alcool cétylstéarylique et d'alcool cétylstéarylique oxyéthyléné (33 motifs OE) 80/20 vendu par la société TENSIA sous la dénomination commerciale DEHSCONET® 390 7 g
- Mélange de mono- et de distéarate de glycérol vendu sous la dénomination commerciale CERASYNTH® SD par la société ISP 2 g
- Alcool cétylique 1,5 g
- Polydiméthylsiloxane vendu sous la dénomination DC200Fluid® par la société DOW CORNING 1,5 g
- Glycérine 15 g
- Conservateurs q.s.
- Eau déminéralisée qsp 100 g

La phase grasse est chauffée à environ 70 - 80 °C jusqu'à fusion complète. On ajoute ensuite sous agitation vigoureuse la phase aqueuse contenant le composé de l'exemple 4 en une seule fois à 80°C. On maintient l'agitation pendant 10 à 15 minutes, puis on laisse refroidir sous agitation modérée jusqu'à environ 40 °C et on ajoute les conservateurs. On obtient ainsi une crème antisolaire jaune clair particulièrement efficace contre les UV-B.

## Revendications

1. Composé répondant à la formule générale (1) suivante : 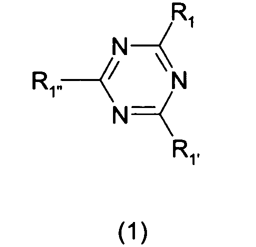 dans laquelle :
R₁, R₁' et R₁", identiques ou différents, désignent :
(i) soit un groupe chromophore répondant à l'une des formules suivantes : 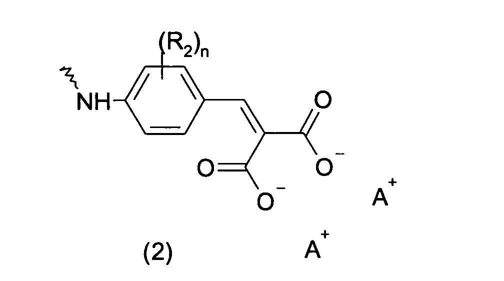 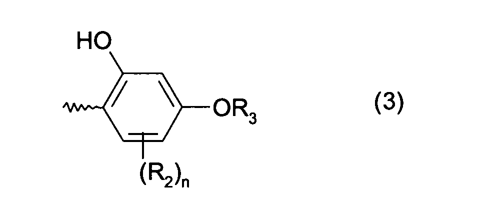 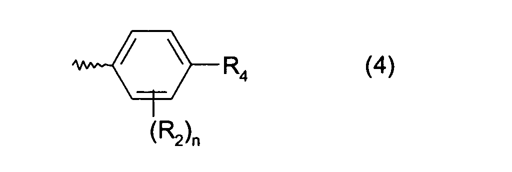 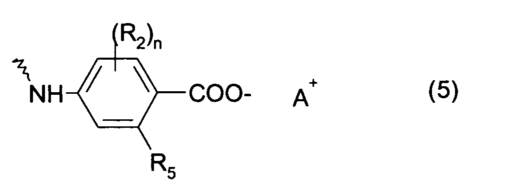 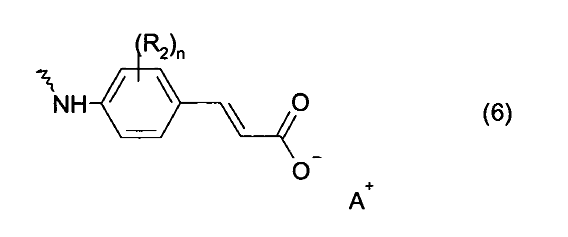 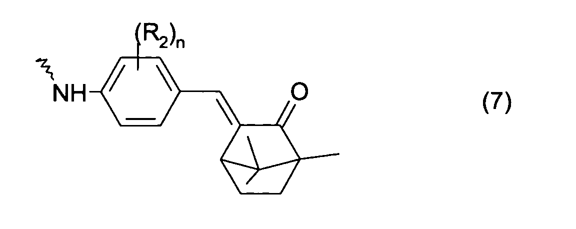 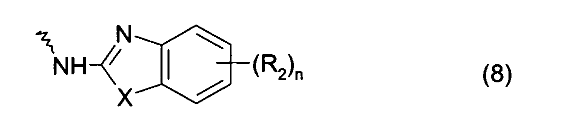
dans lesquelles :
R₂ représente un groupe alkyle en C₁-C₈, linéaire ou ramifié ;
n vaut 0, 1 ou 2,
les radicaux A, identiques ou différents représentent hydrogène ; un cation du type métal alcalin comme sodium ou potassium ; un groupe ammonium ; un radical mono, di- ou tri- alkylammonium en C₁-C₂₀ ; un radical mono, di- ou trialkanolammonium en C₂-C₂₀ ;
R₃ représente un radical alkyle en C₁-C₂₀, linéaire ou ramifié ; un radical alcène en C₂-C₂₀, linéaire ou ramifié ; les dits radicaux pouvant contenir un ou plusieurs atomes d'oxygène, atomes de silicium ou groupes trisiloxanes ;
R₄ représente un radical alkyle en C₁-C₂₀, linéaire ou ramifié ; un radical alcènyle en C₂-C₂₀, linéaire ou ramifié ; un radical alcoxy en C₁-C₂₀, linéaire ou ramifié ; lesdits radicaux pouvant contenir un ou plusieurs atomes d'oxygène, atomes de silicium ou groupes trisiloxanes ;
R₅ représente l'hydrogène ou OH,
X représente O, S ou NR₆ avec R₆ représentant H ou un radical alkyle en C₁-C₄, linéaire ou ramifié ;
sous réserve qu'au moins un des radicaux R₁, R₁' et R₁'' désigne un groupe chromophore de formule (2) et qu'au plus un des radicaux R₁, R₁' et R₁'' désigne un groupe chromophore de formule (3) ;
(ii) OH ; un radical alcoxy en C₁-C₂₀, linéaire ou ramifié ; un radical mono ou dialkylamino en C₁-C₂₀, linéaire ou ramifié; lesdits radicaux pouvant contenir un ou plusieurs atomes d'oxygène, atomes de silicium ou groupes trisiloxanes.

2. Composé selon la revendication 1, pour lequel :
R₁, R₁' et R₁", identiques ou différents, désignent :
(i) soit un chromophore de formule : 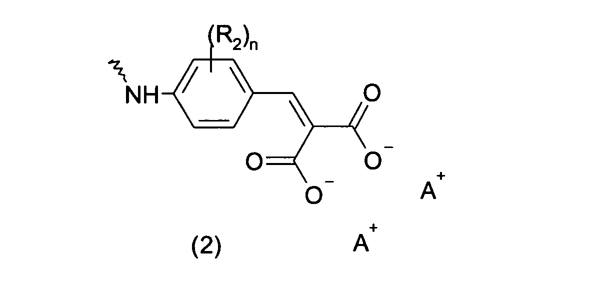 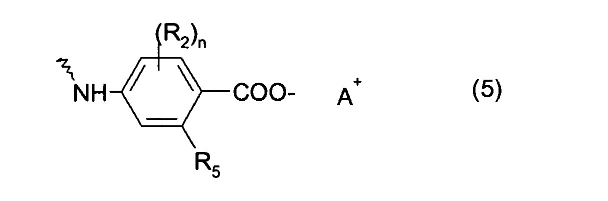 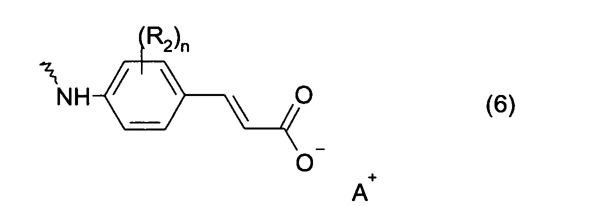 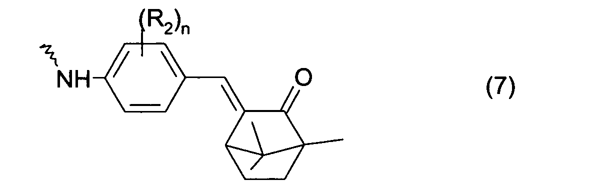
dans lesquelles :
A désigne Na, K ou triéthanolamine ;
n=0;
R₅ désigne hydrogène ou OH ;
(ii) soit un radical OH ; un radical alcoxy en C₁-C₄ ; un radical dialkylamino en C₁-C₄.

3. Composé selon la revendication 1 ou 2, choisi parmi les composés suivants :

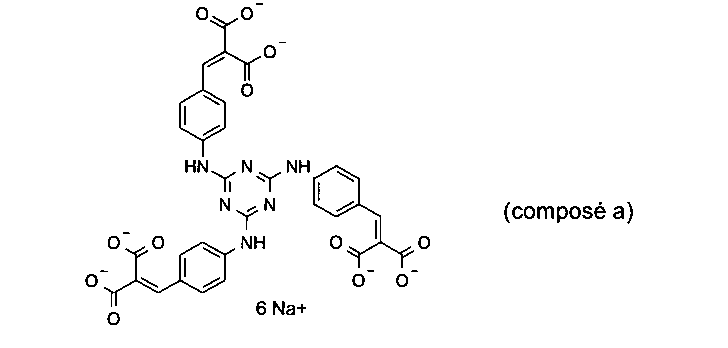

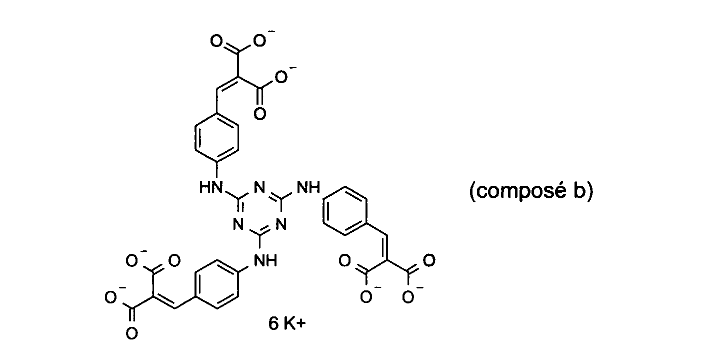

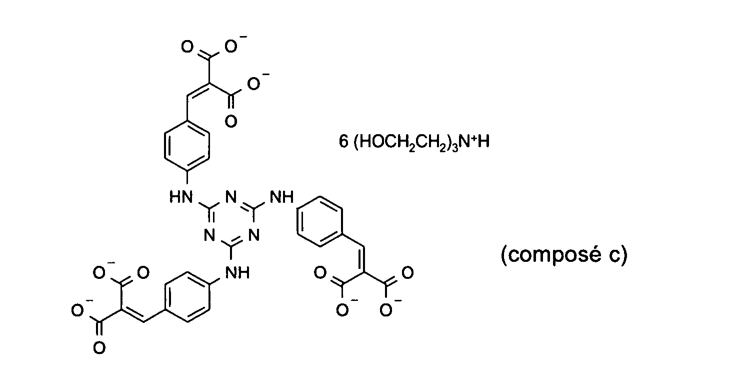

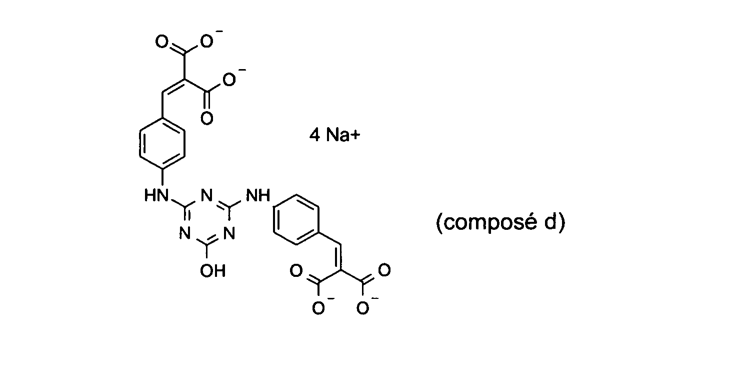

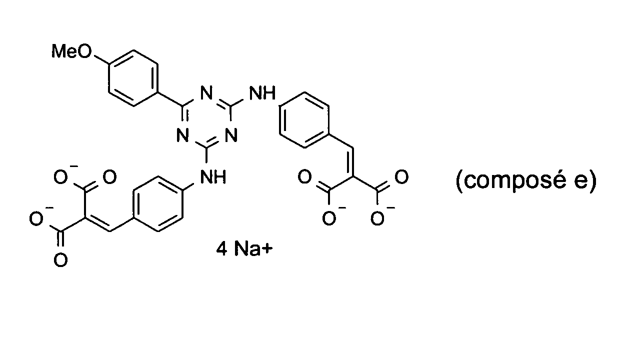

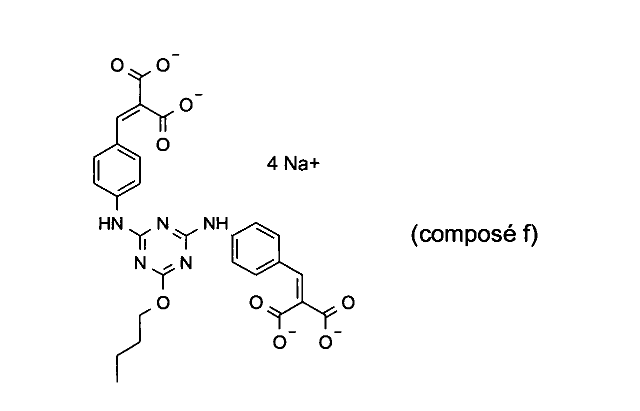

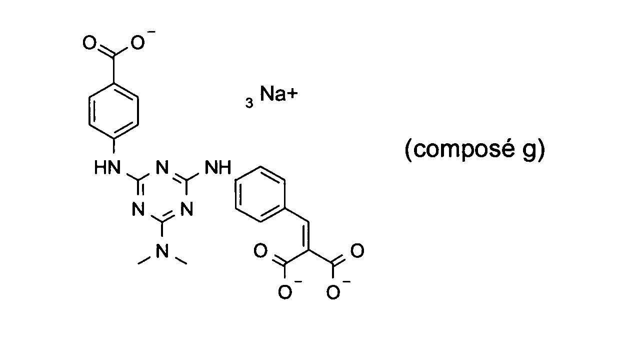

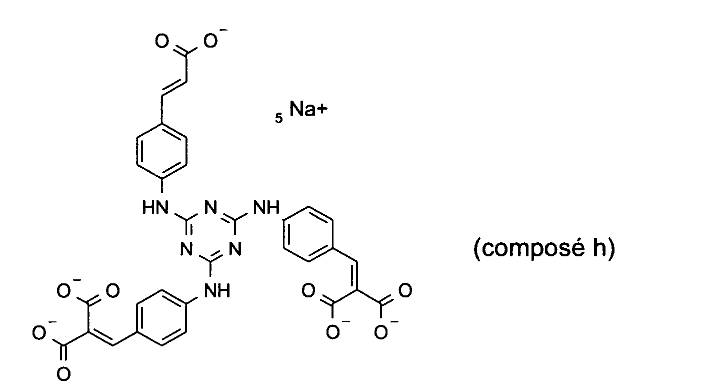

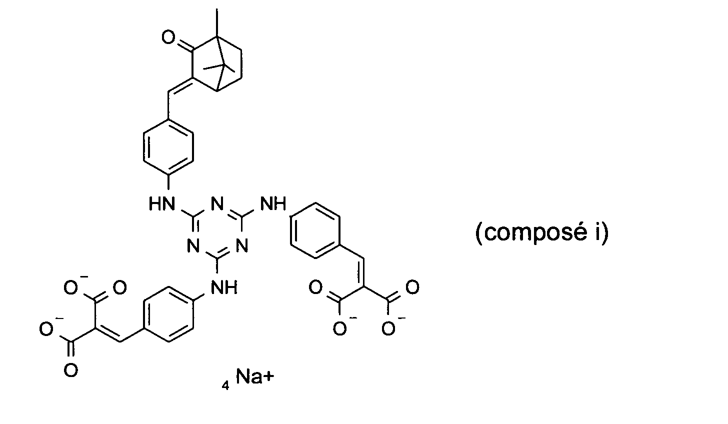

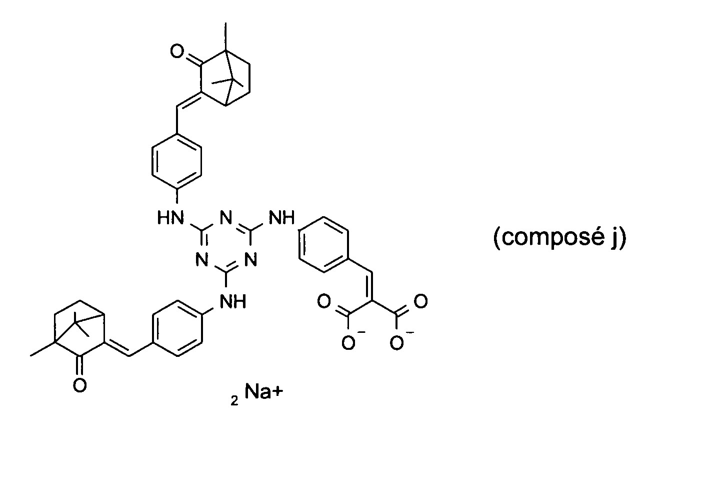

4. Procédé de préparation d'un composé de formule (1) tel que défini dans l'une quelconque des revendications précédentes, qui consiste à faire réagir un aminobenzalmalonate de formule (D) suivante 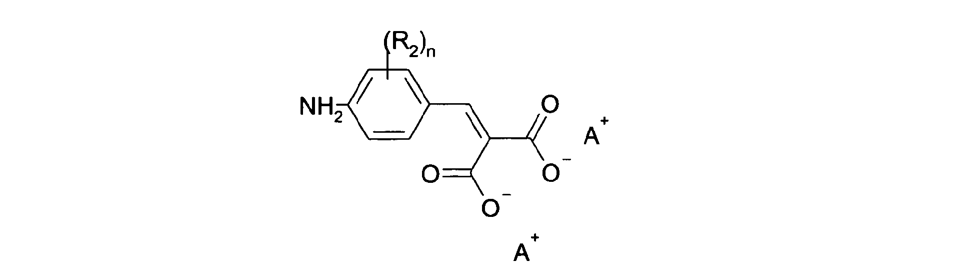 dans laquelle A, R₂, n ont les mêmes significations indiquées dans les revendications précédentes :
(i) lorsque le composé de formule (1) comporte trois groupements de formule (2) , sur un composé de formule (A) suivante : 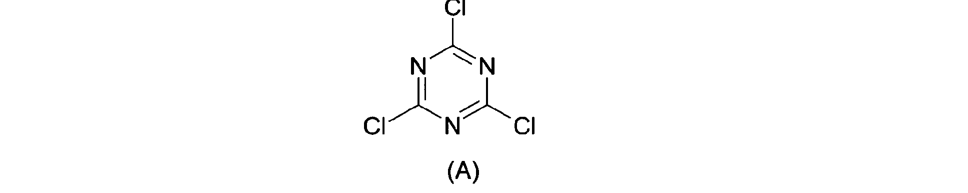
(ii) lorsque le composé de formule (1) comporte un seul groupement de formule (2), sur un composé de formule (B) suivante : 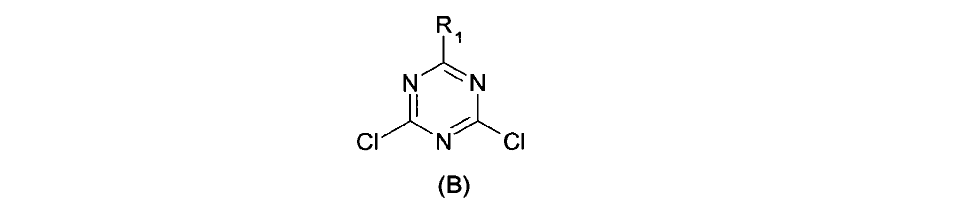
(iii) lorsque le composé de formule (1) comporte deux groupements de formule (2), sur un composé de formule (B) suivante :

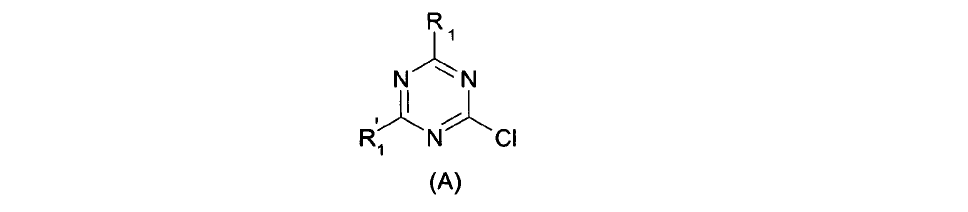

5. Procédé selon la revendication 4, où les conditions de mise en oeuvre sont les suivantes : milieu aqueux homogène ou en suspension et en présence ou pas d'un co-solvant organique, le pH étant ajusté pendant la réaction à un pH n'excédant pas 9,5 et plus particulièrement inférieur à 9,0 et à une température comprise entre +5°C-120°C et plus particulièrement entre +5°C-85°C.
Comme co-csovant organique, on peut utiliser entre autres l'acétone, le THF ou le toluène.
Comme agent basique, on peut utiliser entre autres la soude, la potasse, le bicarbonate ou le carbonate de sodium.

6. Composition cosmétique ou dermatologique, destinée à la photoprotection des matières kératiniques, **caractérisée par le fait qu'**elle contient dans un support cosmétiquement acceptable au moins un composé méthyltrialkylsilane répondant à la formule (I) tel que défini dans l'une quelconque des revendications 1 à 3.

7. Composition selon la revendication 6, **caractérisée par le fait que** le composé de formule (I) est présent dans la composition à une teneur allant de 0,01 à 20 % en poids, par rapport au poids total de la composition, de préférence allant de 0,1 % à 10 % en poids, par rapport au poids total de la composition.

8. Composition selon la revendication 6 ou 7, **caractérisée par le fait que** ledit support cosmétiquement acceptable se présente sous la forme d'une émulsion de type huile-dans-eau ou eau-dans-huile.

9. Composition selon l'une quelconque des revendications 6 à 8, **caractérisée par le fait qu'**elle comprend en outre un ou plusieurs filtres organiques ou inorganiques complémentaires actifs dans l'UV-A et/ou UV-B.

10. Composition selon la revendication 9, **caractérisée par le fait que** lesdits filtres organiques complémentaires sont choisis parmi les anthranilates ; les dérivés cinnamiques ; les dérivés de dibenzoylméthane ; les dérivés salicyliques, les dérivés du camphre ; les dérivés de triazine; les dérivés de la benzophénone ; les dérivés de β,β-diphénylacrytate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres; les dimères dérivés d'α-alkylstyrène; les 4,4-diarylbutadiènes et leurs mélanges.

11. Composition selon la revendication 10, **caractérisée par le fait que** lesdits filtres organiques complémentaires sont choisis parmi
Ethylhexyl Salicylate,
Ethylhexyl Methoxycinnamate
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
2,4,6-tris-(4'-amino benzalmalonate de diisobutyle)-s-triazine Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine et leurs mélanges.

12. Composition selon la revendication 9, **caractérisée par le fait que** les filtres complémentaires inorganiques sont des pigments ou des nanopigments d'oxydes métalliques, enrobés ou non.

13. Composition selon la revendication 12, **caractérisée par le fait que** lesdits pigments ou nanopigments sont choisis parmi les oxydes de titane, de zinc, de fer, de zirconium, de cérium et leurs mélanges, enrobés ou non.

14. Composition selon l'une quelconque des revendications 1 à 13, **caractérisée par le fait qu'**elle comprend en outre au moins un agent de bronzage et/ou de brunissage artificiel de la peau.

15. Composition selon l'une quelconque des revendications 1 à 14, **caractérisée par le fait qu'**elle comprend en outre au moins un adjuvant choisi parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants

16. Composition selon l'une quelconque des revendications 1 à 15, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une huile, d'une poudre, d'un bâtonnet solide, d'une mousse ou d'un spray.

17. Composition selon l'une quelconque des revendications 1 à 15, **caractérisée par le fait qu'**il s'agit d'une composition de maquillage des cils, des sourcils, des ongles ou de la peau et qu'elle se présente sous forme solide ou pâteuse, anhydre ou aqueuse, d'une émulsion, d'une suspension ou d'une dispersion.

18. Composition selon l'une quelconque des revendications 1 à 15, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

19. Utilisation d'un composé de formule (I) tel que défini dans l'une quelconque des revendications 1 à 3 dans une composition cosmétique ou dermatologique comme agent filtrant les radiations UV.

20. Utilisation d'un composé de formule (I) tel que défini dans l'une quelconque des revendications 1 à 3 dans une composition cosmétique comme agent de contrôle de la variation de la couleur de la peau due aux rayonnements UV.

21. Utilisation d'un composé de formule (I) tel que défini dans l'une quelconque des revendications 1 à 3 comme agent photostabilisant de polymères synthétiques ou de verres.
